# FASCICULE DE BREVET EUROPEEN

(11) **EP 0 908 184 B1**
(45) Date de publication et mention de la délivrance du brevet: **05.03.2003**
(21) Numéro de dépôt: 98460042.9
(22) Date de dépôt: 06.10.1998
(51) Int. Cl.: A61K 35/08

(54) **Composition à base d'eau de mer diluée destinée au lavage des fosses nasales**
Zusammensetzung auf Basis von verdünntem Meerwasser zur Behandlung der nasalen Fossae
Diluted seawater based composition for the treatment of the nasal fossae

(30) Priorité: 10.10.1997 FR 9712931
(43) Date de publication de la demande: 14.04.1999
(73) Titulaire: P.N. Gerolymatos S.A., 145 65 Kryoneri Attica (GR)
(72) Inventeur: Maugendre, Renée-Jane, 35400 Saint-Malo (FR)
(74) Mandataire: Maillet, Alain

(56) Documents cités:
- CA-A- 1 313 142
- CA-A- 2 093 573
- FR-A- 2 299 041
- FR-B- 2 735 980
- FR-B- 2 741 535
- FR-B- 2 741 536
- US-A- 4 581 226
- Le compendium suisse des Médicaments (1997) 1085 XP002932344

## Description

La présente invention concerne une composition à base d'eau de mer destinée au lavage des fosses nasales. La composition selon l'invention est notamment destinée à la résorption des oedèmes de la muqueuse nasale, au traitement des sinusites chroniques, des rhinites allergiques et des polyposes nasales.

Les fosses nasales ont pour principales fonctions de réguler le courant d'air inspiratoire, de réchauffer et d'humidifier l'air inspiré, et d'éliminer les éléments étrangers (poussières, germes microbiens).

L'épithélium cilié qui tapisse la muqueuse nasale joue un rôle essentiel dans la défense de celle-ci contre les infections. Les cils vibratiles dudit épithélium sont baignés par un mucus de défense qui les entoure, et ils sont prévus pour faire progresser par leurs battements ledit mucus et les impuretés externes d'avant en arrière, c'est-à-dire vers le pharynx.

Les compositions à base d'eau de mer qui sont connues à l'heure actuelle pour le lavage des fosses nasales sont généralement prévues pour être isotoniques à l'égard de la muqueuse nasale. Chacune de ces compositions est par exemple constituée d'un mélange d'eau de mer et d'eau désionisée, qui est tel que la fraction volumique d'eau de mer dans ledit mélange est d'environ un tiers. Ce mélange est soumis à une filtration destinée à le rendre stérile avant son conditionnement, et la composition finalement obtenue suite à ladite filtration est par exemple utilisée sous la forme d'une bombe aérosol.

On pourra par exemple se reporter au document de brevet français FR-A-2 299 041 pour un exemple de préparation d'une telle composition.

Lesdites compositions isotoniques peuvent également être obtenues par extraction d'une forte proportion d'ions sodium, potassium, chlorure d'un échantillon d'eau de mer brute, par exemple par électrodialyse.

Ces compositions isotoniques sont généralement bien tolérées par la muqueuse nasale et elles n'induisent pas de modifications sensibles du système muco-ciliaire.

Cependant, elles présentent l'inconvénient de ne pas donner des résultats probants pour le traitement efficace d'obstructions nasales sérieuses ou d'oedèmes importants, par exemple.

On a donc cherché à élaborer des compositions hypertoniques qui seraient susceptibles de décongestionner plus efficacement les fosses nasales. Ces compositions sont constituées d'une solution d'eau de mer brute qui est filtrée et stérilisée.

Le document de brevet canadien CA-A-1 313 142 décrit un exemple d'obtention d'une telle composition, ainsi que les effets observés chez des sujets auxquels cet exemple de composition hypertonique a été administré.

Cette composition est essentiellement caractérisée par la région de prélèvement de l'eau de mer qui la constitue, laquelle se situe au large du Canada. Cette région détermine le degré de salinité de ladite composition, ainsi que son pH qui est égal à 7,2.

Un inconvénient majeur de ce type de composition réside, d'une part, dans le phénomène d'intolérance qu'elle engendre pour la muqueuse nasale et, d'autre part, dans la perturbation de la physiologie du système muco-ciliaire de ladite muqueuse.

L'intolérance précitée vis-à-vis de la muqueuse nasale peut notamment se traduire par un réflexe sternutatoire dû à l'irritation des filets nerveux et sensitifs de ladite muqueuse.

Quant à ladite perturbation du système muco-ciliaire, laquelle résulte notamment de la salinité et du pH relativement modéré de ladite composition, elle peut se traduire par un ralentissement, voire une cessation du mouvement de battement des cils vibratiles, ce qui entrave l'écoulement précité du mucus de défense.

Il en résulte une fragilisation de la muqueuse nasale vis-à-vis des impuretés inspirées.

Le but de la présente invention est de proposer une composition à base d'eau de mer destinée au lavage des fosses nasales, ladite composition étant constituée d'un mélange d'eau de mer préalablement filtrée, purifiée et stérilisée et d'eau distillée, qui permette de remédier à cet inconvénient et, par conséquent, d'éviter les effets indésirables qui en résultent suite à son administration.

A cet effet, une composition selon l'invention est telle que la fraction volumique d'eau de mer dans ledit mélange est sensiblement égale à 70 %.

Selon une autre caractéristique de l'invention, la concentration de chlorure de sodium à l'état dissous dans ledit mélange est comprise entre 23 g/l et 25 g/l.

De préférence, ladite concentration de chlorure de sodium est sensiblement égale à 24 g/l.

Selon une autre caractéristique de l'invention, le pH de ladite composition est sensiblement égal à 8,2.

De préférence, la concentration de magnésium à l'état dissous dans ledit mélange est sensiblement égale à 1,04 g/l.

De préférence, la concentration de calcium à l'état dissous dans ledit mélange est sensiblement égale à 0,39 gel.

De préférence, la concentration de potassium à l'état dissous dans ledit mélange est sensiblement égale à 0,25 g/l.

De préférence, la concentration de zinc à l'état dissous dans ledit mélange est sensiblement égale à 0,09 mg/l.

De préférence, la concentration de cuivre à l'état dissous dans ledit mélange est sensiblement égale à 0,04 mg/l.

Une composition selon l'invention est en outre prévue pour être administrée sous la forme de pulvérisations, chaque pulvérisation étant réalisée par nébulisation de microparticules sous l'effet d'une pression active.

Les caractéristiques de l'invention mentionnées ci-dessus, ainsi que d'autres, apparaîtront plus clairement à la lecture de la description suivante d'un exemple de réalisation.

Une composition selon la présente invention est constituée d'un mélange d'eau de mer prélablement filtrée, purifiée, stérilisée et d'eau distillée. Cette composition est telle que la fraction volumique d'eau de mer qui est contenue dans ledit mélange est sensiblement de 70 %.

De préférence, l'eau de mer utilisée pour ladite composition est prélevée en France, dans la baie de Cancale.

La concentration de chlorure de sodium à l'état dissous dans ledit mélange est comprise entre 23 g/l et 25 g/l, et elle est de préférence égale à 24 g/l.

A titre préférentiel, une composition selon l'invention est caractérisée par un pH qui est sensiblement égal à 8,2, c'est-à-dire qui définit un milieu alcalin.

Ladite composition contient en outre les éléments minéraux suivants à l'état dissous avec les concentrations correspondantes:

| | |
|---|---|
| magnésium | 1,04 g/l; |
| calcium | 0,39 g/l; |
| potassium | 0,25 g/l; |
| zinc | 0,09 mg/l; |
| cuivre | 0,04 mg/l. |

Une composition selon l'invention est destinée à être conditionnée dans un flacon pressurisé pourvu d'un embout auto-stérilisant.

De préférence, le mode d'administration de cette composition consiste en des pulvérisations par nébulisation de microparticules disséminées, sous l'effet d'une pression active et dynamique.

On a procédé à des tests cliniques en pathologie sinusienne d'une composition selon l'invention sur des sujets dont l'âge est compris entre 15 et 70 ans. Ces troubles sont tels qu'ils s'accompagnent constamment d'une obstruction nasale, même après mouchage, avant leur traitement avec ladite composition.

Lesdits troubles sont par exemple des rhinites oedémateuses, allergiques, catarrhales, hypertrophiques, vasculaires, des polyposes, etc.

Les contre-indications relatives audit traitement sont, d'une part, les états infectieux aigüs tels que les sinusites aiguës et, d'autre part, les troubles hémostatiques et/ou de l'hémostase.

Le protocole qui a été suivi pour ces tests est le suivant, en référence aux tableaux 1 à 6 figurant ci-après. Ces tableaux concernent 30 sujets représentatifs du point de vue du degré des troubles affectant leurs muqueuses nasales respectives.

Dans un premier temps, on a défini pour chaque sujet présentant un trouble du type précité les signes cliniques qu'il présente (voir tableau 1).

Ces signes cliniques ont été étudiés du point de vue de l'obstruction nasale observée, de ses effets de gêne vis-à-vis de l'endormissement et du sommeil, de la respiration buccale, des troubles de l'olfaction et de la nature de la rhinorrhée accompagnant ladite obstruction nasale.

Chaque fois que cela a été possible, on a quantifié ladite obstruction nasale au moyen d'une échelle de valeurs comprises entre 0 et 10 qui est connue sous le nom d'Echelle Visuelle Analogique (EVA).

Dans un second temps, on a procédé pour chacun desdits sujets à des examens généraux consistant essentiellement en une rhinoscopie antérieure des fosses nasales (par nasofibroscopie), de manière à pouvoir caractériser précisément l'oedème et/ou l'inflammation de la muqueuse. On a également défini la nature d'éventuelles déformations anatomiques et sécrétions affectant ladite muqueuse.

Les résultats de ces examens généraux sont consignés dans le tableau 2.

Dans un troisième temps, on a administré à chaque sujet une composition selon l'invention de la manière suivante.

On a procédé à deux nébulisations par fosse nasale le matin, le midi et le soir, et ce pendant 15 jours. On notera que ces nébulisations permettent une diffusion de la composition sur une surface de muqueuse d'aire élevée.

En cas d'obstruction nasale très importante, on a procédé pendant environ les cinq premier jours du traitement à une pulvérisation locale d'une composition connue sous le nom Aturgyl®, quelques minutes avant chaque nébulisation d'une composition selon l'invention. Ces pulvérisations complémentaires n'ont en aucun cas été poursuivies après le huitième jour de traitement.

Suivant les troubles affectant les sujets à traiter, on a associé l'administration d'une composition selon l'invention à celle d'antibiotiques.

Par contre, on n'a administré en aucun cas des produits du type mucolytiques, anti-inflammatoires non stéroïdiens (AINS) ou corticoïdes.

On a identifié au tableau 3 les sujets qui ont été traités par de l'Aturgyl® et/ou un ou plusieurs antibiotiques, en plus de ladite composition selon l'invention.

Dans un quatrième temps, on a énuméré les résultats observés la fin du traitement précité, tant du point de vue des signes cliniques, des examens généraux susmentionnés que du point de vue du résultat global observé (voir tableaux 4, 5 et 6, respectivement).

Certains paramètres figurant dans les tableaux de résultats ci-dessous sont définis par les symboles +, 2+ et 3+. Ces symboles correspondent respectivement à des quantités réduites, assez importantes et très importantes.

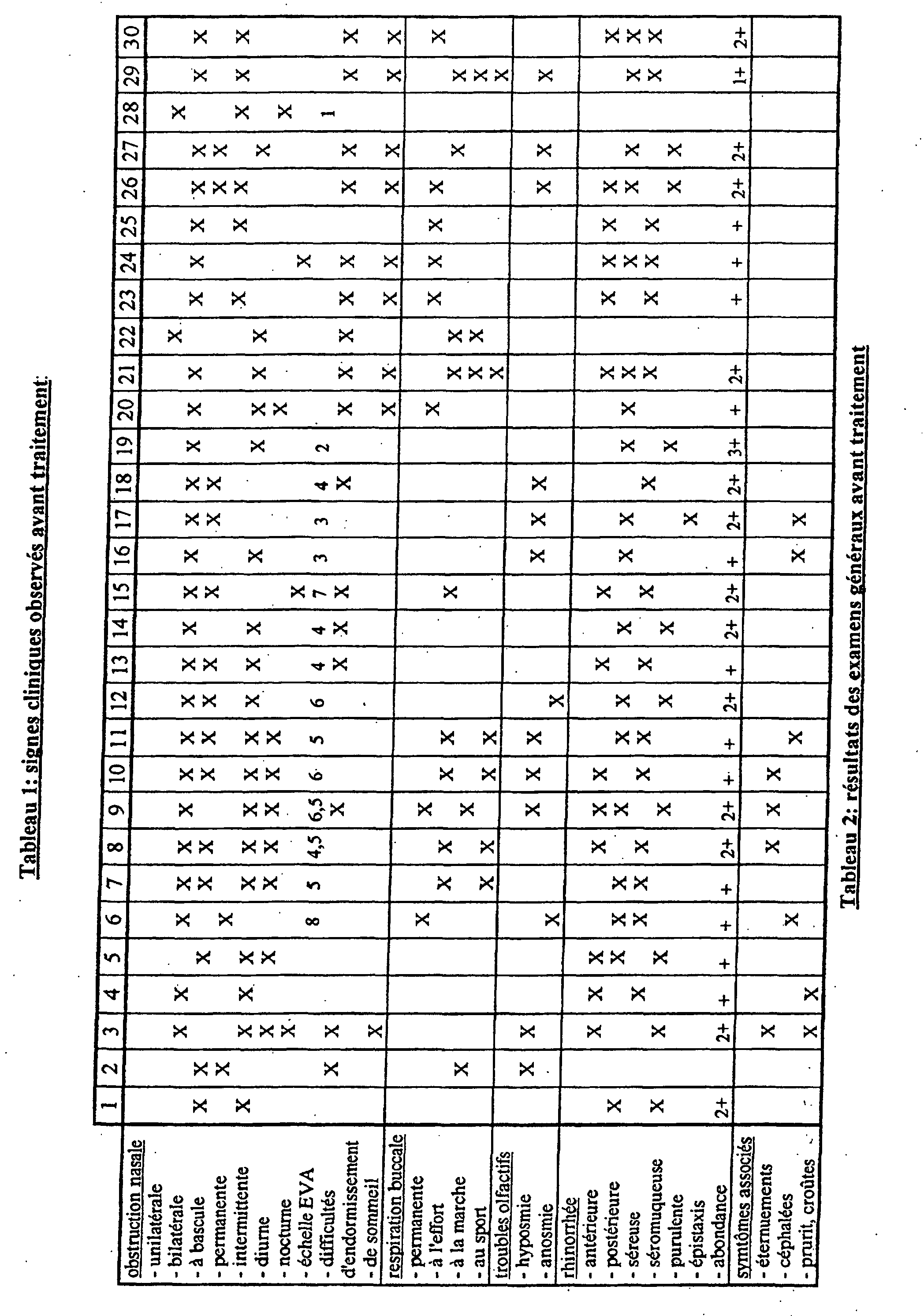

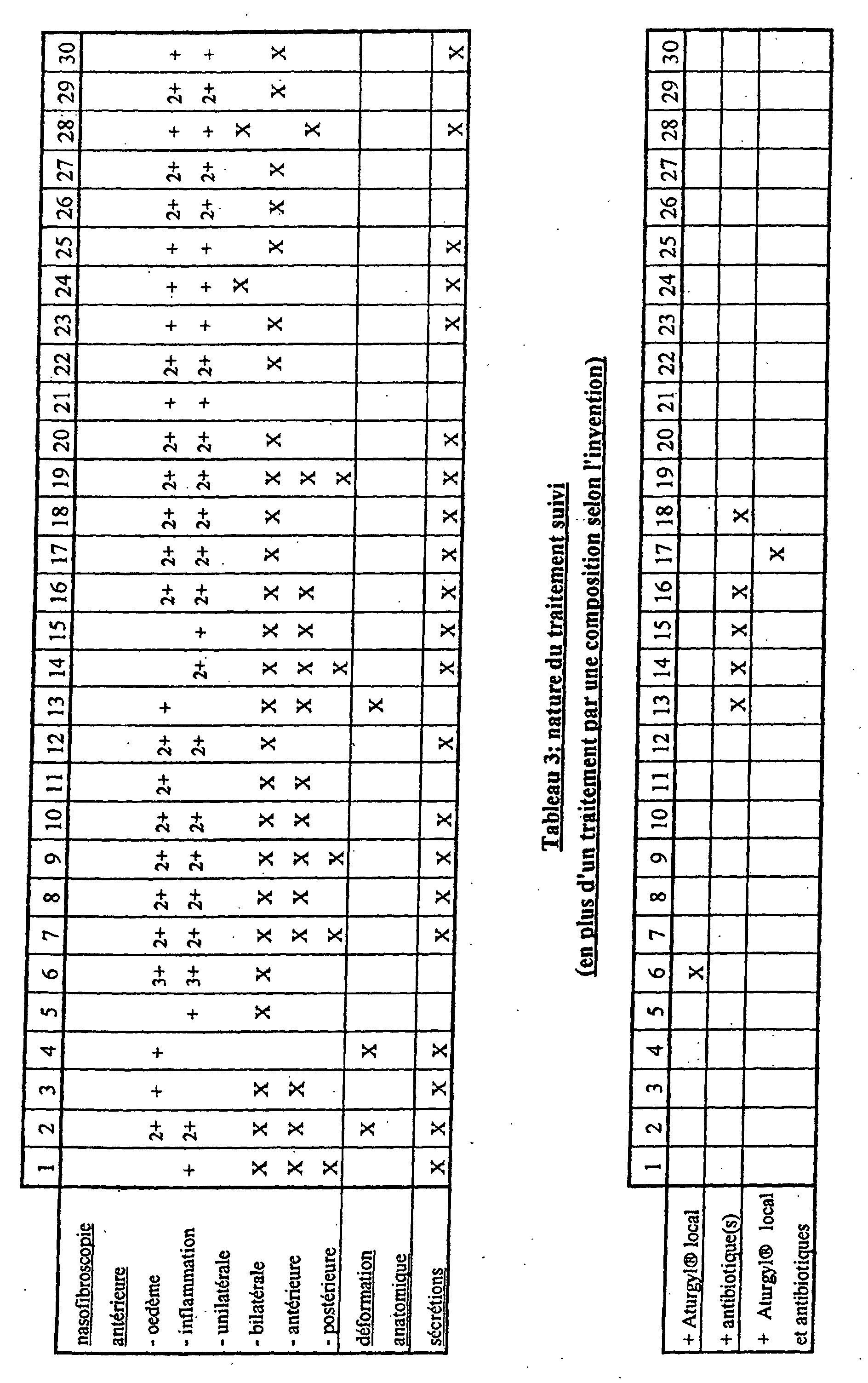

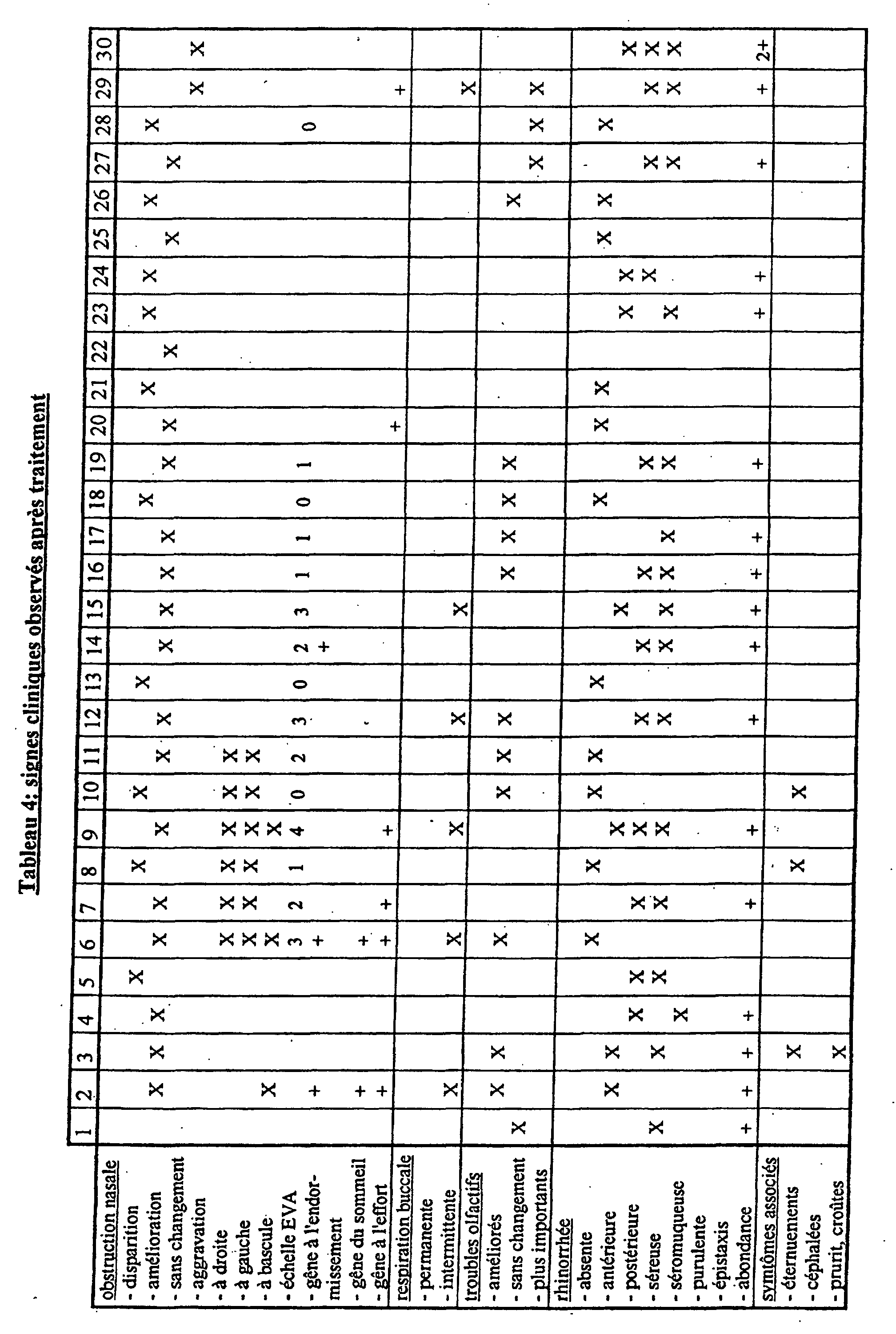

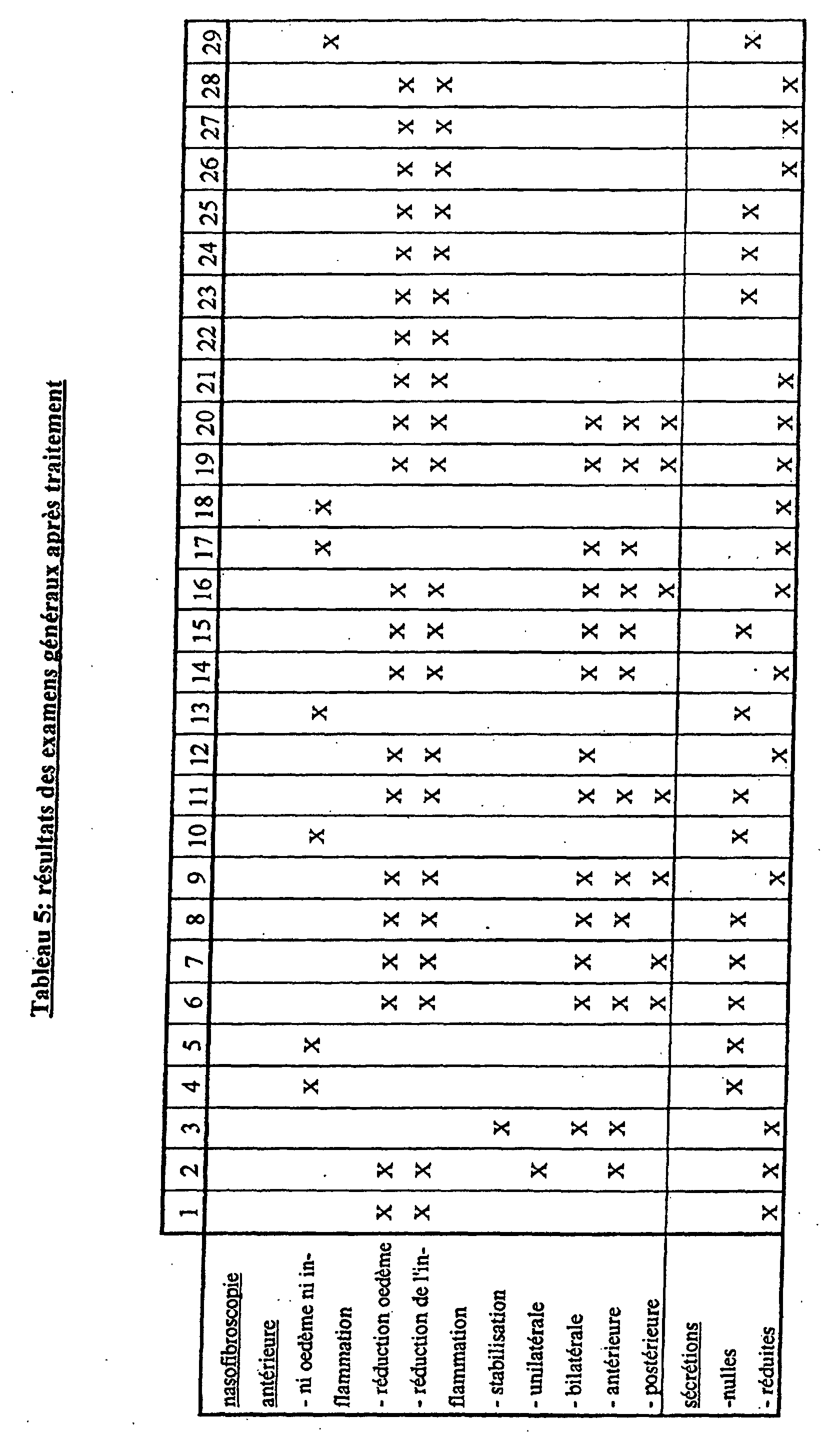

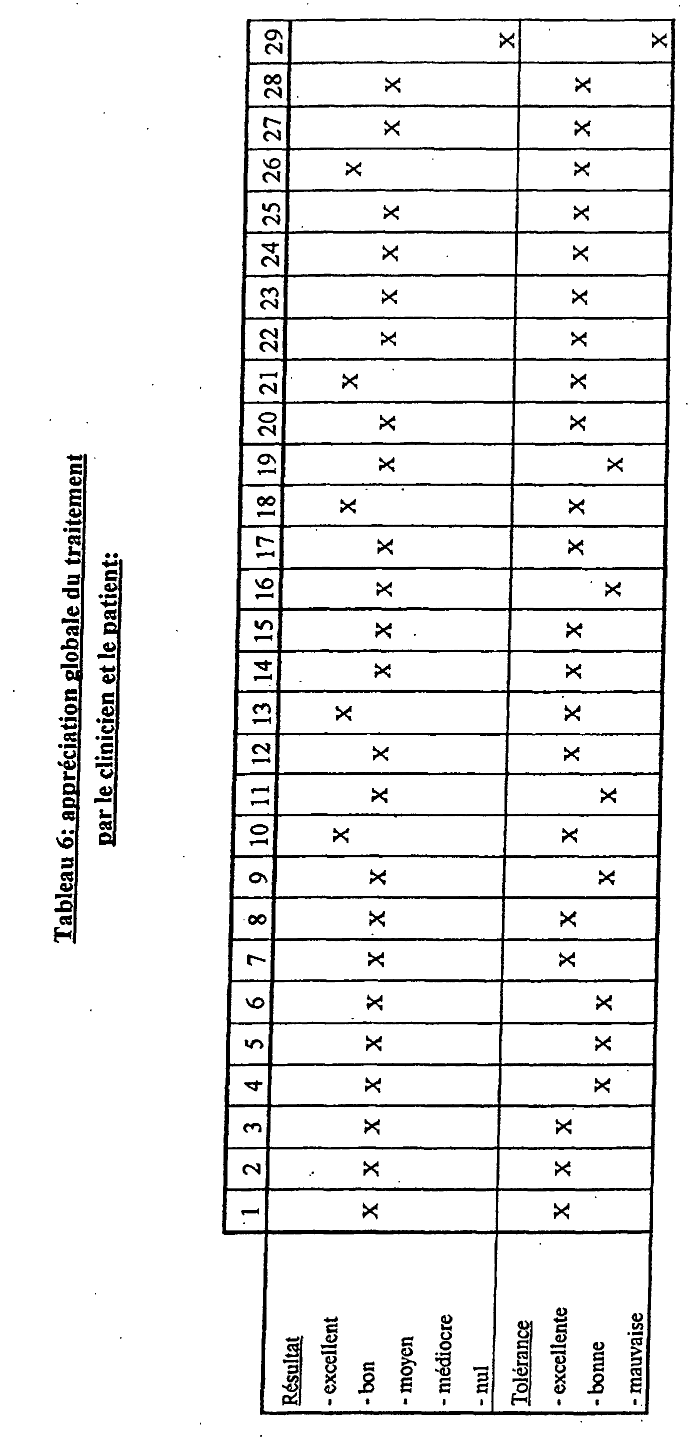

Les résultats qui sont exposés ci-dessus montrent que l'administration d'une composition selon l'invention permet en général de décongestionner efficacement les fosses nasales, en résorbant les oedèmes et les obstructions qui les affecten (voir échelle EVA), notamment.

Cette efficacité s'explique par le fait que ladite composition optimise le phénomène d'osmose par appel de l'eau contenue en excès dans les cellules de la muqueuse.

On notera que ce drainage des sécrétions par variation de la pression osmotique ne provoque ni irritation de la muqueuse, ni modifications physiologiques de l'épithélium cilié, contrairement aux compositions hypertoniques de l'art antérieur. En particulier, la fonction dudit épithélium qui est de faire s'écouler vers le pharynx le mucus de défense est préservée.

On notera également que le pH alcalin de la composition selon l'invention, qui est sensiblement de 8,2, favorise l'éviction des virus dans la muqueuse nasale.

## Revendications

1. Composition à base d'eau de mer destinée au lavage des fosses nasales, ladite composition étant constituée d'un mélange d'eau de mer préalablement filtrée, purifiée et stérilisée et d'eau distillée, **caractérisée en ce que** la fraction volumique d'eau de mer dans ledit mélange est sensiblement égale à 70 %.

2. Composition à base d'eau de mer destinée au lavage des fosses nasales selon la revendication 1, **caractérisée en ce que** la concentration de chlorure de sodium à l'état dissous dans ledit mélange est comprise entre 23 g/l et 25 g/l.

3. Composition à base d'eau de mer destinée au lavage des fosses nasales selon la revendication 2, **caractérisée en ce que** ladite concentration de chlorure de sodium est sensiblement égale à 24 g/l.

4. Composition à base d'eau de mer destinée au lavage des fosses nasales selon une des revendications précédentes, **caractérisée en ce que** son pH est sensiblement égal à 8,2.

5. Composition à base d'eau de mer destinée au lavage des fosses nasales selon une des revendications 2 à 4, **caractérisée en ce que** la concentration de magnésium à l'état dissous dans ledit mélange est sensiblement égale à 1,04 g/l.

6. Composition à base d'eau de mer destinée au lavage des fosses nasales selon une des revendications 2 à 5, **caractérisée en ce que** la concentration de calcium à l'état dissous dans ledit mélange est sensiblement égale à 0,39 g/l.

7. Composition à base d'eau de mer destinée au lavage des fosses nasales selon une des revendications 2 à 6, **caractérisée en ce que** la concentration de potassium à l'état dissous dans ledit mélange est sensiblement égale à 0,25 g/l.

8. Composition à base d'eau de mer destinée au lavage des fosses nasales selon une des revendications 2 à 7, **caractérisée en ce que** la concentration de zinc à l'état dissous dans ledit mélange est sensiblement égale à 0,09 mg/l.

9. Composition à base d'eau de mer destinée au lavage des fosses nasales selon une des revendications 2 à 7, **caractérisée en ce que** la concentration de cuivre à l'état dissous dans ledit mélange est sensiblement égale à 0,04 mg/l.

## Claims

1. Composition based on seawater intended for the lavage of the nasal fossae, the said composition consisting of a mixture of initially filtered, purified and sterilised sea water and distilled water, **characterised in that** the volume fraction of salt water in the said mixture is approximately equal to 70 %.

2. Composition based on sea water intended for the lavage of the nasal fossae according to claim 1, **characterised in that** the concentration of sodium chloride in the dissolved state in the said mixture is between 23 g/l and 25 g/l.

3. Composition based on seawater intended for the lavage of the nasal fossae according to claim 2, **characterised in that** the said concentration of sodium chloride is approximately equal to 24 g/l.

4. Composition based on seawater intended for the lavage of the nasal fossae according to one of the proceeding claims, **characterised in that** its pH is approximately equal to 8.2.

5. Composition based on seawater intended for the lavage of the nasal fossae according to one of claims 2 to 4, **characterised in that** the concentration of magnesium dissolved in the said mixture is approximately equal to 1.04 g/l.

6. Composition based on seawater intended for the lavage of the nasal fossae according to one of claims 2 to 5, **characterised in that** the concentration of calcium dissolved in the said mixture is approximately equal to 0.39 g/l.

7. Composition based on seawater intended for the lavage of the nasal fossae according to claims 2 to 6, **characterised in that** the concentration of potassium in the dissolved state in the said mixture is approximately equal to 0.25 g/l.

8. Composition based on seawater intended for the lavage of the nasal fossae according to one of claims 2 to 7, **characterised in that** the concentration of zinc in the dissolved state in the said mixture is approximately equal to 0.09 mg/l.

9. Composition based on seawater, intended for the lavage of the nasal fossae according to claims 2 to 7, **characterised in that** the concentration of copper in the dissolved state in the said mixture is approximately equal to 0.04 mg/l.

## Patentansprüche

1. Zusammensetzung auf der Basis von Meerwasser zur Spülung der Nasenhöhlen, bestehend aus einer Mischung aus zuvor gefiltertem, gereinigtem und sterilisiertem Meerwasser und destilliertem Wasser, **dadurch gekennzeichnet, dass** der Volumenanteil des Meerwassergehalts der Mischung im Wesentlichen 70 % beträgt.

2. Zusammensetzung auf der Basis von Meerwasser zur Spülung der Nasenhöhlen nach Anspruch 1, **dadurch gekennzeichnet, dass** das in der Mischung gelöste Natriumchlorid in einer Konzentration von 23 g/l bis 25 g/l vorliegt.

3. Zusammensetzung auf der Basis von Meerwasser zur Spülung der Nasenhöhlen nach Anspruch 2, **dadurch gekennzeichnet, dass** das Natriumchlorid in einer Konzentration von etwa 24 g/l vorliegt.

4. Zusammensetzung auf der Basis von Meerwasser zur Spülung der Nasenhöhlen nach einem der vorstehenden Ansprüche, **dadurch gekennzeichnet, dass** sie einen pH-Wert von etwa 8,2 aufweist.

5. Zusammensetzung auf der Basis von Meerwasser zur Spülung der Nasenhöhlen nach einem der Ansprüche 2 bis 4, **dadurch gekennzeichnet, dass** das in der Mischung gelöste Magnesium in einer Konzentration von etwa 1,04 g/l vorliegt.

6. Zusammensetzung auf der Basis von Meerwasser zur Spülung der Nasenhöhlen nach einem der Ansprüche 2 bis 5, **dadurch gekennzeichnet, dass** das in der Mischung gelöste Kalzium in einer Konzentration von etwa 0,39 g/l vorliegt.

7. Zusammensetzung auf der Basis von Meerwasser zur Spülung der-Nasenhöhlen nach einem der Ansprüche 2 bis 6 **dadurch gekennzeichnet, dass** das in der Mischung gelöste Kalium in einer Konzentration von etwa 0,25 g/l vorliegt.

8. Zusammensetzung auf der Basis von Meerwasser zur Spülung der Nasenhöhlen nach einem der Ansprüche 2 bis 7, **dadurch gekennzeichnet, dass** das in der Mischung gelöste Zink in einer Konzentration von etwa 0,09 mg/l vorliegt.

9. Zusammensetzung auf der Basis von Meerwasser zur Spülung der Nasenhöhlen nach einem der Ansprüche 2 bis 7, **dadurch gekennzeichnet, dass** das in der Mischung gelöste Kupfer in einer Konzentration von etwa 0,04 mg/l vorliegt.
